# EUROPEAN PATENT SPECIFICATION

(11) **EP 1 001 839 B1**
(45) Date of publication and mention of the grant of the patent: **18.06.2003**
(21) Application number: 98937137.2
(22) Date of filing: 24.07.1998
(51) Int. Cl.: B01D 63/02, B01D 63/10, B01D 69/10

(54) **METHOD OF HOLLOW FIBER FILTRATION**
VERFAHREN ZUR HOHLFASERN FILTRATION
PROCEDE DE FILTRATION A FIBRES CREUSES

(30) Priority: 06.08.1997 US 907307
(43) Date of publication of application: 24.05.2000
(73) Proprietor: Genentech, Inc., South San Francisco, CA 94080-4990 (US)
(72) Inventor: VAN REIS, Robert, D., Redwood City, CA 94062 (US)
(74) Representative: Kiddle, Simon John
(86) International application number: US9815476
(87) International publication number: WO99007458

(56) References cited:
- DATABASE WPI Week 9413 Derwent Publications Ltd., London, GB; AN 94-106079 XP002078769 & JP 06 055038 A (MITSUBISHI RAYON CO LTD) , 1 March 1994
- PATENT ABSTRACTS OF JAPAN vol. 96, no. 011, 29 November 1996 & JP 08 187086 A (ASAHI CHEM IND CO LTD), 23 July 1996
- PATENT ABSTRACTS OF JAPAN vol. 13, no. 238, 5 June 1989 & JP 01 051075 A (ASAHI CHEM IND CO LTD), 27 February 1989
- PATENT ABSTRACTS OF JAPAN vol. 96, no. 5, 31 May 1996 & JP 08 023976 A (ASAHI CHEM IND CO LTD), 30 January 1996

## Description

### FIELD OF THE INVENTION

The present invention relates to processes useful for improved hollow fiber membrane filtration-mediated purification and separation of molecules from mixtures of molecules, wherein those molecules may be of biological interest. More specifically, the present invention provides a process useful for controlling transmembrane pressure along the length of a hollow fiber membrane during filtration of complex macromolecular mixtures.

### BACKGROUND OF THE INVENTION

Several methods are currently available for separating and purifying molecules of biological interest, such as proteins, from complex mixtures thereof. One of the most well known processes for separating and purifying molecules of interest is membrane filtration; a process which separates dissolved and suspended solutes in solutions based upon their size. In its simplest form, membrane filtration involves the passage of a solution containing the molecule of interest through a filtration membrane having pores of a defined size. Solutes larger than the membrane pore size are retained, while solutes smaller than the pore size pass through the membrane with the solvent, thereby effectively separating the larger solutes from the smaller solutes.

Such membrane filtration processes generally fall within the categories of reverse osmosis, microfiltration and ultrafiltration depending upon the pore size of the membrane employed. Conventionally, reverse osmosis employs filtration membranes which are capable of retaining salts and other low molecular weight solutes whereas microfiltration, or microporous filtration, employs membranes in the 0.1 to 10 micron pore size range, typically used to retain colloids and microorganisms. Ultrafiltration, on the other hand, employs filtration membranes rated for retaining solutes between approximately 1 and 1000 kDa in molecular weight and has proven useful for processing up to thousands of liters of solution per hour, specifically for protein concentration and the removal of low molecular weight contaminants from large-scale recombinant protein preparations.

The JP-A-06055038 describes a hollow fiber filter module comprising a mat of hollow fiber membranes layered onto the surface of a flexible screen.

While the ultrafiltration process has proven useful for a variety of applications, limitations do exist on the degree of protein purification achievable by the process. These limitations are due mainly to the well known phenomena of concentration polarization, fouling and wide membrane pore size distribution. See, e.g., Porter, ed., *Handbook of Industrial Membrane Technology* (Noyes Publications, Park Ridge, N.J., 1990), pp.164-173. As a result of these limitations, difficulties have been encountered in the large scale resolution of macromolecular mixtures. See Michaels, "Fifteen Years of Ultrafiltration: Problems and Future Promises of an Adolescent Technology", in Anthony R. Cooper, ed., *Ultrafiltration Membranes and Applications, Polymer Science and Technology,* 13 (Plenum Press, N.Y., 1979), pp. 1-16, at p. 9. Thus, the efficiency of ultrafiltration in large scale complex macromolecular mixture separations has proven to be less than desirable.

In an attempt to overcome the above described limitations of the ultrafiltration process, however, modifications of the process have been developed. One important such modification is the development of the tangential flow filtration (TFF) process. In the TFF process, a solution is passed over a filtration membrane at a high velocity and tangential to the plane of the membrane surface so as to enhance transport of membrane-retained solutes away from the membrane surface and back into the retentate. One major problem in tangential flow filtration, however, is the creation of a differential transmembrane pressure (TMP) gradient along the length of the filtration membrane. Generally, the TMP at the portion of the filtration membrane nearest the feed inlet is very high and it progressively decreases along the length of the filtration membrane as the fluid moves away from the feed inlet toward the retentate outlet end of the filtration device, where the TMP may be very low. As higher feed-side velocities are employed to combat concentration polarization and fouling, the differential TMP gradient over the length of the membrane increases.

This differential TMP gradient causes a number of drawbacks. For example, one drawback of a differential TMP gradient in the filtering passage is that the operational conditions of the filter vary along the entire length thereof. As such, the TMP over a very large percentage of the filter surface will vary considerably from that which provides for optimal filtration efficiency.

Efforts have been made, however, to control the TMP gradient over the length of the filtration membrane so as to provide for a substantially constant TMP across the entire filter surface. For example, U.S. Patent No. 5,256,294, issued on October 26, 1993 to van Reis, describes a high performance tangential flow filtration (HPTFF) process which is performed under certain conditions of flux, where the flux ranges from 5% to up to 100% of the transition point flux, and TMP, where the TMP is held at a range in the pressure dependent region of the flux versus TMP curve, namely, at a range that is no greater than the TMP value at the transition point. In this process, the filtrate is recirculated in a co-current mode. By this mechanism, when the filtrate flow on the filtrate side of the membrane is such that the pressure drop on the filtrate side of the membrane is similar to that on the retentate side of the membrane, a substantially constant TMP is maintained over the entire length of the membrane, thereby allowing one to obtain an optimal TMP over the entire length of the membrane. Recirculation of the filtrate in parallel to the feed flow is also disclosed in U.S. Patent No. 4,105,547 by Sandblom as providing a means for obtaining a substantially constant TMP across the entire length of the filtration membrane.

However, while co-current filtrate recirculation has proven to be useful for controlling the TMP gradient in flat sheet filtration membrane systems, it has been less than effective in systems employing hollow fiber filtration membranes. This is because the co-current filtrate recirculation rate has to be very high, often higher than the feed rate, in order to accomplish an equal TMP across the length of the hollow fibers because of the very low resistance on the shell side of a hollow fiber cartridge. As such, other methods have been employed in hollow fiber filtration systems in attempts to maintain substantially constant TMPs over the entire length of the hollow fiber. For example, U.S. Patent No. 5,525,144 by Gollan discloses a hollow fiber filtration cartridge that has filtrate flow restrictions embedded into the intracartridge potting material which serve as obstacles to filtrate flow, thereby controlling the pressure drop along the filtrate side of the hollow fiber and, in turn, reducing variations in TMP along the length of the fiber. However, because the filtrate flow restrictions are spaced in such a way as to create intracartridge "subcompartments", the pressure drop on the filtrate side of the membrane is not linear, thereby making it difficult to control TMP variance in an exact manner. Moreover, such hollow fiber cartridges are difficult and expensive to manufacture. As such, there is a need for hollow fiber filtration methods which provide for a linear pressure drop on the filtrate side of the membrane.

Therefore, it is an object of the present invention to provide processes for improved filtration in hollow fiber filtration systems.

### SUMMARY OF THE INVENTION

In accordance with the present invention, processes are provided which are useful for improved tangential flow-mediated purification and separation of molecules from mixtures of molecules, wherein those molecules may be of biological interest. More specifically, the present invention provides methods which employ a hollow fiber membrane filtration module useful for separating species of interest from a mixture, wherein said filtration module comprises (a) a filter cartridge shell and (b) a hollow fiber filter element contained within the filter cartridge shell. As employed in the presently described filtration module, the hollow fiber filter element comprises a mat of hollow fibers which is physically layered onto the surface of a screen, wherein the hollow fibers of the filter element are configured into a bundle and the screen occupies at least a portion of the void volume between the hollow fibers. By occupying at least a portion of the void volume between the hollow fibers, the screen functions to control the pressure drop on the filtrate side of the hollow fiber membrane, thereby allowing one to obtain a substantially constant transmembrane pressure along the entire length of the hollow fibers in the filtration module. In place of the screen, however, in another embodiment of the present invention, a portion of the void volume between the hollow fibers may be occupied by a material which functions to weave the substantially parallel hollow fibers into a mat. This material may also provide a significant restriction to flow on the filtrate side of the membrane, thereby allowing one to maintain a substantially constant TMP across the entire length of the hollow fibers in the filtration module. The hollow fiber filtration modules may be employed either with or without co-current recirculation of the filtrate on the filtrate side of the hollow fibers.

In certain preferred embodiments, the hollow fiber mat is a monolayer mat wherein the hollow fibers of the mat may optionally be interwoven with a material that provide restriction to flow on the filtrate side of the fibers. In other embodiments, the hollow fibers employed may be used for microfiltration or ultrafiltration, in the latter where the fibers are sufficient for retaining solutes from about 1 to 1000 kDa in size. The filtration module is useful for separating a variety of different species from mixtures, wherein in preferred embodiments, those species are cells, proteins, viruses, and the like.

Another aspect of the present invention is directed to a method of making a hollow fiber filtration module useful for separating species of interest from a mixture, wherein the method comprises:
(a) providing a mat of hollow fibers;
(b) physically layering the mat of hollow fibers onto a screen to provide a hollow fiber mat/screen complex;
(c) configuring the hollow fiber mat/screen complex into a form wherein the hollow fibers are in a bundle and the screen occupies at least a portion of the void volume between the hollow fibers such that the flow of filtrate between the hollow fibers is restricted and the transmembrane pressure is substantially constant along the entire length of the hollow fibers in the module, thereby providing a hollow fiber filter element; and
(d) inserting the hollow fiber filter element into a filter cartridge shell, wherein the hollow fiber filter element is positioned within the filter cartridge shell so as to allow filtration of a fluid through the hollow fiber filter element.
In a preferred embodiment, the step of configuring the hollow fiber mat/screen complex comprises rolling that complex into a substantially cylindrical shape. The hollow fiber mat may optionally be permanently attached to the screen.

Yet another aspect of the present invention is directed to a method of separating a species of interest from at least one component of a mixture as set out in claims 1 and 10, wherein the method comprises filtering a fluid containing the species of interest through one of the hollow fiber membrane filtration modules described above, wherein at least a portion of the pore sizes of the hollow fibers present in the filtration module are sufficiently small so as to not allow passage of the species of interest yet are sufficiently large to allow passage of one or more other components. In preferred embodiments, the species of interest is a recombinantly produced protein, a cell or a cellular component Often, the pore sizes of the hollow fibers employed are rated for retaining solutes in the range of about 1 to 1000 kDa in size.

Other aspects of the present invention will become evident to those of ordinary skill in the art upon a reading of the present specification.

### BRIEF DESCRIPTION OF THE DRAWINGS

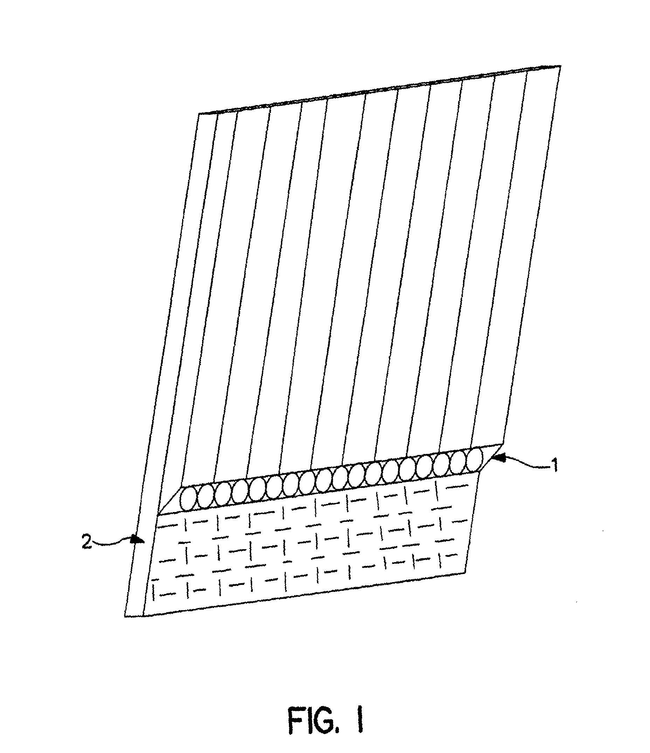

Figure 1 shows one embodiment of the present invention wherein a monolayer hollow fiber filter mat **1** is physically layered onto a screen **2** such that when the hollow fibers are configured into a bundle to form a hollow fiber filter element, the screen **2** will occupy at least a portion of the void volume between the hollow fibers in the bundle.

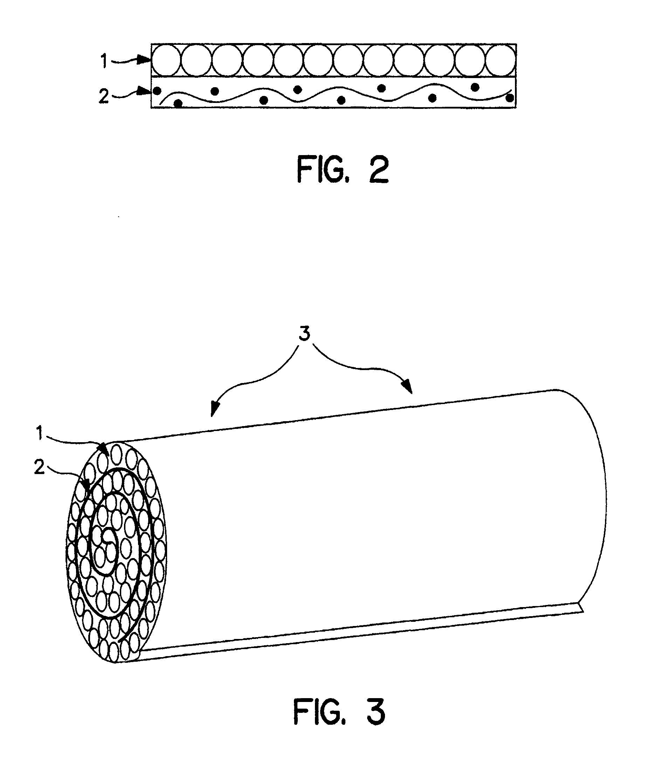

Figure 2 shows a cross-sectional view of a monolayer hollow fiber filter mat **1** which is physically layered onto a screen **2**.
Figure 3 shows a hollow fiber filter element **3** which has been rolled into a substantially cylindrical shape wherein the hollow fibers of the hollow fiber mat **1** and the screen **2** upon which the hollow fiber mat is physically layered are arranged in an internal substantially spiral structure.

### DETAILED DESCRIPTION OF THE PREFERRED EMBODIMENTS

### A. Definitions

As used herein, a "hollow fiber membrane filtration module" means a filtration apparatus which is useful for separating species of interest from a mixture and which comprises both (1) a filter cattridge shell and (2) a hollow fiber filter element contained within that cartridge shell. As used herein, the expression "hollow fiber filter element" and grammatical equivalents thereof refer to the portion of the filtration module which is inserted into the filter cartridge shell and which comprises a mat of hollow fibers which are physically layered onto the surface of a support screen. In other embodiments, however, the hollow fibers of the hollow fiber filter element may be configured substantially parallel and interwoven with a material which occupies at least a portion of the void volume between the fibers, thereby providing a restriction to flow on the filtrate side of the fibers. In the latter embodiment, the hollow fiber filter element may optionally comprise a screen.

As described in detail below, once the mat of hollow fibers is physically layered onto the surface of the screen, it is conditioned into a shape where the hollow fibers are configured into a bundle and where the screen takes up or occupies at least a portion of the void volume between the hollow fibers. Preferably, the hollow fiber mat/screen complex is rolled into a substantially cylindrical shape which has an internal structure wherein the hollow fibers take a multi-layered spiral shape wherein each layer of hollow fibers in the spiral is separated by a layer of the screen. Further detail as to the components of the hollow fiber filter element will be provided below.

As used herein, the expression "filter cartridge shell" means the component of the complete filtration module which serves to encompass and support the hollow fiber filtration element. The cartridge shell may take many different forms, preferably cylindrical, and sizes, depending upon the form and size of the hollow fiber filter element. Many cartridge shells are well known in the art and are commercially available.

As used herein, the expression "physically layered" when used in regard to the hollow fiber mat and screen means that the hollow fiber mat is placed directly upon a screen to provide a means for increasing the resistance to filtrate flow between the hollow fibers when present in a hollow fiber filter element, thereby significantly reducing the pumping capacity required to maintain a substantially constant TMP along the length of the hollow fibers. The hollow fiber mat may then be physically and/or irreversibly attached to the screen by means well known to those of ordinary skill in the art, although such is not critical to the present invention.

Generally, the hollow fiber filter element is maintained within the cartridge shell of the filtration module by conventional potting technology which is well known in the art. The filtration module will generally have feed inlet and retentate and filtrate outlets through which a fluid may be introduced into the module prior to filtration and recovered from the module after filtration, respectively. Various different filtration module designs are well known in the art and may be employed in the filtration module of the present invention.

As used herein, the term "species" generally means a particle(s) or molecule(s) that is to be separated from a solution or suspension in a fluid, e.g., a liquid. The species are separated from the fluid and, in most instances, from other particles or molecules in the fluid. The size of the species of interest to be separated will determine the pore size of the hollow fibers to be employed. Preferably, the species are biological entities of natural biological or biochemical origin or produced by biological or biochemical processes. Examples of preferred species include, without being limiting of the invention, mammalian cells and microorganisms such as bacteria, fungi and yeast, as well as species of suitable size for ultrafiltration, for example, polypeptides, proteins, either naturally or recombinantly produced, cellular components, DNA, colloids, mycoplasm, endotoxins, viruses, bacteria, carbohydrates, and other biological molecules or interest, whether glycosylated or not. In one embodiment, the species of interest are biological molecules having a molecular weight of at least about 1000 daltons and most preferably are polypeptides or proteins. In yet another embodiment, the herein described hollow fiber filtration module employs fibers having a pore size of from 0.1 to I micron or larger for use in microfiltration.

As used herein, a "mixture" of species refers to a fluid solution or suspension which contains two or more different species. The types of species present in any mixture may differ greatly, wherein a mixture may contain, for example, various different polypeptides, buffers, salts, cells and cell components, and the like. Mixtures may be complex, wherein a large number of different species are represented, or relatively simple, where only a small number of different species are represented.

As used herein, the expression "transmembrane pressure" or "TMP" refers to the pressure differential gradient that is applied along the length of a hollow fiber filtration membrane to cause fluid and filterable solutes to flow through the pores of the filter.

As used herein, the expression "substantially constant" as applied to TMP refers to a situation where the TMP at the feed inlet is no more than about 2-fold greater than the TMP at the retentate outlet. It is to be understood, however, that for various applications the ratio of TMP at the feed inlet versus TMP at the retentate outlet of the hollow fiber filtration module may vary considerably, but this ratio is usually less than about 1.8, preferably less than about 1.5 and more preferably less than about 1.3.

As used herein, the expression "same pore size" as applied to the hollow fiber filtration membranes refers to membranes which are rated or sold as having the same pore size, even though the actual pore sizes of the fibers may vary somewhat.

### B. Modes for Carrying Out the Invention

In its broadest aspect, the hollow fiber filtration method of the present invention is useful for separating species of interest from a mixture of different species, wherein the filtration module comprises a filter cartridge shell and a hollow fiber filter element contained within the filter cartridge shell. In one embodiment, the hollow fiber filter element which is inserted into the filter cartridge shell comprises at least two components, i.e., (1) a mat of hollow fibers which is physically layered onto the surface of (2) a screen. The presence of the screen serves to occupy at least a portion of the void volume between the hollow fibers, thereby providing resistance to filtrate flow during filtration, thereby providing a substantially constant TMP across the entire length of the hollow fibers at a lower co-current flow rate.

In another embodiment, the screen as described above may optionally be replaced with a material which functions to weave the hollow fibers together and hold them in a substantially parallel configuration. Because the material which weaves the fibers together (i.e., the interweaving material) functions to occupy at least a portion of the void volume between the hollow fibers, this material may also provide a significant restriction to flow on the filtrate side of the fibers, thereby allowing one to maintain a substantially constant TMP across the entire length of the filtration module. Materials which can serve to weave the hollow fibers together are well known in the art and will be chosen such that they do not adversely effect the species of interest to be filtered from a solution. Interweaving materials generally occupy at least about 5% of the void volume between the hollow fibers, usually from about 5% to about 99% of the void volume between the fibers, preferably from about 15% to about 99% of the void volume and more preferably from about 50% to about 99% of the void volume. The mat of interwoven hollow fibers obtained is referred to herein as the "interwoven hollow fiber mat".

As to the hollow fibers which are incorporated into the filtration module of the present invention, various types of hollow fibers may be employed. For example, hollow fibers which find use in the present invention include hollow fibers formed from various materials based on cellulose, polyolefins, polyvinyl alcohol, PMMA, polysulfones, and the like. Other hollow fibers which find use herein include hollow fibers which are formed from materials such as polyethylene and polypropylene, such fibers being capable of being easily woven into fabrics. No particular limitation is placed upon the pore size of the hollow fibers employed, nor the porosity, fiber wall thickness and outer diameter of the fiber used, so long as they serve as a filtering membrane for the application desired. For the purification of a recombinantly produced protein from a complex mixture, generally pore sizes which are rated for retaining solutes between approximately 1 and 1000 kDa in molecular weight are preferred. Pore sizes of from about 0.1 to 10 microns are generally employed for the purification of biomolecules from cellular debris and intact cells. The hollow fibers employed herein may be prepared using methods which are well known to those of ordinary skill in the art and/or are commercially available.

The hollow fibers employed in the present invention are typically formed into a mat, which is preferably a monolayer mat, however, multiple layer hollow fiber mats may also find use herein. The fibers of a hollow fiber filter element are typically configured in a substantially parallel fashion as is well known and routinely employed in the art. The hollow fibers of a mat may optionally be woven together by a material which is placed substantially perpendicularly to the length of the fibers to form an interwoven hollow fiber mat as described above. Techniques for preparing interwoven hollow fiber mats having widely-spaced polymeric threads interwoven perpendicularly to the hollow fibers are well known to those of ordinary skill in the art. The hollow fiber mat may take virtually any shape and/or size depending upon the desired application, however square or rectangular shapes are generally preferred.

Once prepared, the mat of hollow fibers as described above may then be physically layered onto a surface of a screen, wherein the screen functions to provide a means for increasing the resistance to filtrate flow between the hollow fibers when present in a hollow fiber filter element, thereby significantly reducing the pumping capacity required to maintain a substantially constant TMP along the length of the hollow fibers. The screen is generally comprised of a perforated or fenestrated material such as a porous mesh or other screen-like material and may comprise any suitable material which provides for increased resistance to filtrate flow between the hollow fibers and which does not adversely react with the fluid to be filtered or any component of interest contained therein. The precise composition employed to produce the screen, as well as other parameters considered in the preparation of an operational hollow fiber filtration element, are well known and may be readily selected by those of ordinary skill in the art for particular end-uses. However, non-limiting examples of screen components which find use in the present invention include plastics, synthetic polymeric materials, metals including, for example, aluminum or stainless steel, and the like. Preferably, the screen takes a size and shape that is similar to that of the hollow fiber mat which is physically layered thereon, however, such is not critical to the invention and the size and shape of the screen may vary greatly depending upon the desired application. One embodiment of a hollow fiber mat **1** which is physically layered onto the surface of a screen **2** is shown in Fig. 1. Fig. 2 shows a cross-sectional view of a hollow fiber mat **1** which is physically layered onto the surface of a screen **2**.

Once the mat of hollow fibers is physically layered onto the screen and optionally attached thereto as described above or once the interwoven hollow fiber mat is obtained, a hollow fiber filter element is prepared by conditioning the hollow fiber mat/screen complex or interwoven mat into a form where the hollow fibers are configured into a bundle and the screen or interweaving material occupies at least a portion of the void volume between the hollow fibers. By "bundle" is meant that the fibers of the mat are brought into close physical proximity. Preferably, the hollow fiber mat/screen complex or the interwoven hollow fiber mat is rolled into a substantially cylindrical shape which has an internal substantially spiral structure. Therefore, in one embodiment, internal to the hollow fiber filter element are hollow fibers arranged in a substantially spiral shape wherein the hollow fibers may be separated by a layer of screen or interweaving material. One embodiment of a hollow fiber/screen complex which has been configured into a substantially spiral shape is shown in Fig. 3.

By "substantially spiral" is meant that internal to the filter element, there are multiple substantially circular shaped layers of hollow fibers which begin from a central internal point and extend outwardly in a circular fashion. Therefore, unlike in conventional hollow fiber bundles, much of the void volume between the hollow fibers is replaced in the presently described element with a porous screen-like material or interweaving material which provides significant resistance to filtrate flow therethrough, thereby increasing the resistance and, in turn, significantly reducing the pumping capacity required to maintain a substantially constant TMP along the length of the hollow fibers. The hollow fiber mat/screen complex or interwoven hollow fiber mat may be rolled into a substantially cylindrical shape as tightly or as loosely as desired, thereby controlling the amount of void volume present in the hollow fiber filter element.

Once prepared, the substantially spiral shaped hollow fiber filter elements of the present invention may further be enclosed within a cartridge shell or housing unit such that a complete hollow fiber filtration module is formed. Housing unit or cartridge shell configurations, materials therefor, and method for their preparation, are well known to, and appreciated by, those of ordinary skill in the art. Generally, housing units and cartridge shells are prepared from inert materials so as to ensure that there is no adverse interaction between the housing unit or cartridge shell and the fluid to be filtered or any component of interest contained therein. The spiral shaped hollow fiber filter elements described above may be prepared and inserted into a hollow fiber cartridge shell and potted in place using conventional methodology, thereby providing a complete hollow fiber filtration cartridge or module.

The precise housing unit or cartridge shell composition employed and size parameters of the housing unit, as well as other parameters considered in the preparation of an operational hollow fiber filtration module, are well known and may be readily selected by those of ordinary skill in the art for particular end-uses.

The hollow fiber filtration processes as described herein may be employed in a variety of applications, including, but not limited to, the separation and purification of one or more proteins from a complex mixture of different proteins, especially during large scale recombinant protein production. The filtration modules and processes of the present invention are applicable for separation and purification of a wide range of biological molecules, e.g., proteinaceous products of fermentation of natural or genetically engineered microorganisms, polypeptides, antibodies, cellular secretions, colloids, mycoplasm, endotoxins, viruses, bacteria, amino acids, DNA, RNA, carbohydrates, and the like.

The present invention also finds use for separating cellular debris from whole cells. For example, in continuous perfusion cultures, it would be desirable to not only exchange media components and remove products, but also to remove cellular debris that would otherwise accumulate during the run. In such a case, the hollow fiber pore size employed would be close to that of intact cells, thereby allowing cellular debris to flow into the filtrate while whole cells are retained in the retentate.

The presently described processes may also be employed for separating inorganic contaminants from various mixtures containing organic and/or inorganic molecules. For example, proteins present in a complex mixture may be effectively separated and purified away from salts, buffers, and the like, which are commonly found in crude protein preparations. Components found in mixtures may also be effectively concentrated using the presently claimed invention.

Filtration as described above may be performed by passing an appropriate fluid of interest through the hollow fiber filtration element or complete filter module prepared as described above. Methods and techniques for doing so are well known in the art.

The present invention has a number of advantages over the existing prior art. First, by reducing or eliminating the requirement for filtrate recirculation to maintain a substantially constant TMP over the entire length of the filter element, the filtration system is more economical and less complex that other known filtrations systems. The use of a porous screen or an interweaving material in the hollow fiber filter element controls the pressure drop on the filtrate side of the membrane, thereby allowing one to maintain a substantially constant TMP over the length of the filtration cartridge.

The foregoing description details specific methods which can be employed to practice the present invention. Having detailed such specific methods, those skilled in the art will well enough know how to devise alternative reliable devices and methods at arriving at the same information in using the fruits of the present invention. Thus, however detailed the foregoing may appear in text, it should not be construed as limiting the overall scope thereof; rather, the ambit of the present invention is to be determined only by the lawful construction of the appended claims.

## Claims

1. A method of separating a species of interest from at least one component of a mixture, said method comprising filtering a fluid containing said species of interest through a hollow fiber membrane filtration module, wherein the hollow fiber membrane filtration module comprises:
(a) a filter cartridge shell; and
(b) a hollow fiber filter element contained within said filter cartridge shell, said hollow fiber filter element comprising a mat of hollow fibers physically layered onto the surface of a flow-restricting screen, wherein the hollow fibers of said filter element are configured into a bundle and said flow-restricting screen occupies at least a portion of the void volume between said hollow fibers such that the flow of filtrate between said hollow fibers is restricted and the transmembrane pressure is substantially constant along the entire length of the hollow fibers in said module; and
wherein at least a portion of the pore sizes of the hollow fibers present in said module are sufficiently small so as to not allow passage of said species of interest yet are sufficiently large to allow passage of said at least one component.

2. The method according to claim 1, wherein said mat of hollow fibers is a monolayer mat of hollow fibers.

3. The method according to claim 1 or claim 2, wherein said hollow fibers have a pore size of from about 1 to 1000 kDa.

4. The method according to any one of claims 1 to 3, wherein said species of interest are selected from the group consisting of cells, proteins and viruses.

5. The method according to claim 4, wherein said cells are mammalian cells.

6. The method according to claim 4, wherein said cells are *E. coli* cells.

7. The method according to any one of the preceding claims, wherein said species of interest is a component of an *E. coli* lysate.

8. The method according to claim 4, wherein said proteins are recombinantly expressed proteins.

9. The method according to any one of the preceding claims, wherein said hollow fiber filter element has a substantially cylindrical shape.

10. A method of separating a species of interest from at least one component of a mixture, said method comprising filtering a fluid containing said species of interest through a hollow fiber membrane filtration module, wherein the hollow fiber membrane filtration module comprises:
(a) a filter cartridge shell; and
(b) a flow-restricting mat of hollow fibers contained within said filter cartridge shell, wherein said mat comprises hollow fibers configured substantially in parallel and a material interwoven together with said hollow fibers such that the interwoven material occupies at least about 5% of the void volume between said hollow fibers, thereby restricting the flow of filtrate between said hollow fibers and maintaining a substantially constant transmembrane pressure along the entire length of the hollow fibers in said module; and
wherein at least a portion of the pore sizes of the hollow fibers present in said module are sufficiently small so as to not allow passage of said species of interest yet are sufficiently large to allow passage of said at least one component.

11. The method according to claim 10, wherein said material occupies from about 15% to 99% of the void volume between said hollow fibers.

12. The method according to claim 10 or claim 11, wherein said hollow fibers have a pore size of from about 1 to 1000 kDa.

13. The method according to any one of claims 10 to 12, wherein said species of interest are selected from the group consisting of cells, proteins and viruses.

14. The method according to any one of the preceding claims, wherein said species of interest is a recombinantly expressed protein.

15. The method according to any one of the preceding claims, wherein said species of interest is a cell or a cell component.

16. The method according to claims 10 or 11, wherein said at least a portion of the pore sizes are in the range of about 1 to 1000 kDa.

17. The method according to any one of the preceding claims which further comprises the step of co-currently recirculating the filtrate on the filtrate side of said hollow fibers.

18. A method of making a hollow fiber filtration module useful for separating species of interest from a mixture, said method comprising:
(a) providing a mat of hollow fibers;
(b) physically layering said mat of hollow fibers onto a flow-restricting screen to provide a hollow fiber mat/screen complex;
(c) configuring said hollow fiber mat/screen complex into a form wherein said hollow fibers are in a bundle and said flow-restricting screen occupies at a portion of the void volume between said hollow fibers such that the flow of filtrate between the hollow fibers is restricted and the transmembrane pressure is substantially constant along the entire length of the hollow fibers in the module, thereby providing a hollow fiber filter element; and
(d) inserting said hollow fiber filter element into a filter cartridge shell, wherein said hollow fiber filter element is positioned within said filter cartridge shell so as to allow filtration of a fluid through said hollow fiber filter element.

19. The method according to claim 18, wherein the step of configuring comprises rolling said hollow fiber mat/screen complex into a substantially cylindrical shape.

20. The method according to claim 18 or claim 19, wherein said mat of hollow fibers is a monolayer mat.

21. The method according to any one of claims 18 to 20, wherein the step of physically layering comprises permanently adhering said mat of hollow fibers to said screen.

## Patentansprüche

1. Verfahren zum Abtrennen einer Spezies von Interesse von zumindest einer Komponente eines Gemisches, wobei das Verfahren das Filtrieren eines Fluids, das die Spezies von Interesse enthält, durch ein Hohlfasermembran-Filtrationsmodul umfasst, worin das Hohlfasermembran-Filtrationsmodul Folgendes umfasst:
(a) eine Filterpatronenhülle; und
(b) ein Hohlfaser-Filterelement, das in der Filterpatronenhülle enthalten ist, wobei das Hohlfaser-Filterelement eine Matte aus Hohlfasern umfasst, die lagenförmig auf der Oberfläche einer Siebs mit Drosselwirkung aufgelegt ist, worin die Hohlfasern des Filterelements als Bündel konfiguriert sind und das Sieb mit Drosselwirkung zumindest einen Abschnitt des Hohlraumvolumens zwischen den Hohlfasern einnimmt, so dass der Filtratstrom zwischen den Hohlfasern eingeschränkt ist und der Transmembrandruck über die gesamte Länge der Hohlfasern im Modul im Wesentlichen konstant ist; und
worin zumindest ein Teil der Porengrößen der im Modul vorhandenen Hohlfasern ausreichend gering ist, damit sie den Durchtritt der Spezies von Interesse nicht zulassen, aber ausreichend groß, um den Durchtritt der zumindest einen Komponente zuzulassen.

2. Verfahren nach Anspruch 1, worin die Hohlfasermatte eine einlagige Hohlfaser-Matte ist.

3. Verfahren nach Anspruch 1 oder 2, worin die Hohlfasern eine Porengröße von etwa 1 bis 1.000 kDa aufweisen.

4. Verfahren nach einem der Ansprüche 1 bis 3, worin die Spezies von Interesse aus der aus Zellen, Proteinen und Viren bestehenden Gruppe ausgewählt sind.

5. Verfahren nach Anspruch 4, worin die Zellen Säugetierzellen sind.

6. Verfahren nach Anspruch 4, worin die Zellen E.coli-Zellen sind.

7. Verfahren nach einem der vorangegangenen Ansprüche, worin die Spezies von Interesse eine Komponente eines E.coli-Lysats ist.

8. Verfahren nach Anspruch 4, worin die Proteine rekombinant exprimierte Proteine sind.

9. Verfahren nach einem der vorangegangenen Ansprüche, worin das Hohlfaser-Filterelement eine im Wesentlichen zylindrische Gestalt aufweist.

10. Verfahren zum Abtrennen einer Spezies von Interesse von zumindest einer Komponente eines Gemisches, wobei das Verfahren das Filtrieren eines Fluids, das die Spezies von Interesse enthält, durch ein Hohlfasermembran-Filtrationsmodul umfasst, worin das Hohlfasermembran-Filtrationsmodul Folgendes umfasst:
(a) eine Filterpatronenhülle; und
(b) eine Hohlfasermatte mit Drosselwirkung, die in der Filterpatronenhülle enthalten ist, worin die Matte Hohlfasern, die im Wesentlichen parallel konfiguriert sind, sowie ein Material umfasst, das mit den Hohlfasern verflochten ist, so dass das verflochtene Material zumindest etwa 5 % des Hohlraumvolumens zwischen den Hohlfasern einnimmt, so dass der Filtratstrom zwischen den Hohlfasern eingeschränkt ist und ein im Wesentlichen konstanter Transmembrandruck über die gesamte Länge der Hohlfasern im Modul beibehalten wird; und
worin zumindest ein Teil der Porengrößen der im Modul vorhandenen Hohlfasern ausreichend gering ist, damit sie den Durchtritt der Spezies von Interesse nicht zulassen, aber ausreichend groß, um den Durchtritt der zumindest einen Komponente zuzulassen.

11. Verfahren nach Anspruch 10, worin das Material etwa 15 bis 99 % des Hohlraumvolumens zwischen den Hohlfasern einnimmt.

12. Verfahren nach Anspruch 10 oder 11, worin die Hohlfasern eine Porengröße von etwa 1 bis 1.000 kDa aufweisen.

13. Verfahren nach einem der Ansprüche 10 bis 12, worin die Spezies von Interesse aus der aus Zellen, Proteinen und Viren bestehenden Gruppe ausgewählt sind.

14. Verfahren nach einem der vorangegangenen Ansprüche, worin die Spezies von Interesse ein rekombinant exprimiertes Protein ist.

15. Verfahren nach einem der vorangegangenen Ansprüche, worin die Spezies von Interesse eine Zelle oder eine Zellkomponente ist.

16. Verfahren nach Anspruch 10 oder 11, worin der zumindest eine Teil der Porengrößen im Bereich von etwa 1 bis 1.000 kDa liegt.

17. Verfahren nach einem der vorangegangenen Ansprüche, das weiters den Schritt des gleichzeitigen Rezirkulierens des Filtrats auf der Filtratseite der Hohlfasern umfasst.

18. Verfahren zur Herstellung eines Hohlfaser-Filtrationsmoduls, das zum Abtrennen von Spezies von Interesse von einem Gemisch geeignet ist, wobei das Verfahren Folgendes umfasst:
(a) das Bereitstellen einer Hohlfasermatte;
(b) das physikalische Aufschichten der Hohlfasermatte auf ein Sieb mit Drosselwirkung, um einen Hohlfasermatten/Sieb-Komplex bereitzustellen;
(c) das Konfigurieren des Hohlfasermatten/Sieb-Komplexes in eine Form, worin die Hohlfasern als Bündel vorliegen und das Sieb mit Drosselwirkung einen Teil des Hohlraumvolumens zwischen den Hohlfasern einnimmt, so dass der Filtratstrom zwischen den Hohlfasern eingeschränkt ist und der Transmembrandruck über die gesamte Länge der Hohlfasern im Modul im Wesentlichen konstant ist, wodurch ein Hohlfaser-Filterelement bereitgestellt wird; und
(d) das Einführen des Hohlfaser-Filterelements in eine Filterpatronenhülle, worin das Hohlfaser-Filterelement innerhalb der Filterpatronenhülle so angeordnet wird, dass die Filtation eines Fluids durch das Hohlfaser-Filterelement ermöglicht wird.

19. Verfahren nach Anspruch 18, worin der Schritt des Konfigurierens das Zusammenrollen des Hohlfasermatten/Sieb-Komplexes zu einer im Wesentlichen zylindrischen Gestalt umfasst.

20. Verfahren nach Anspruch 18 oder 19, worin die Hohlfasermatte eine einschichtige Matte ist.

21. Verfahren nach einem der Ansprüche 18 bis 20, worin der Schritt des physikalischen Aufschichtens das permanente Verkleben der Hohlfasermatte mit dem Sieb umfasst.

## Revendications

1. Procédé de séparation d'une espèce à laquelle on s'intéresse d'au moins un constituant d'un mélange, ledit procédé comprenant la filtration d'un fluide dontenant ladite espèce à laquelle on s'intéresse à travers un module de filtration à membrane à fibres creuses, dans lequel le module de filtration à membrane à fibres creuses comporte :
(a) une enveloppe de cartouche à filtre ; et
(b) un élément de filtre à fibres creuses contenu dans ladite enveloppe de cartouche à filtre, ledit élément de filtre à fibres creuses comportant un mât de fibres creuses stratifiées physiquement sur la surface d'un tamis restreignant l'écoulement, dans lequel les fibres creuses dudit élément de filtre sont configurées en un faisceau et ledit tamis de restriction d'écoulement occupe au moins une partie du volume vide entre lesdites fibres creuses afin que l'écoulement d'un filtrat entre lesdites fibres creuses soit restreint et que la pression à travers la membrane soit sensiblement constante sur toute la longueur des fibres creuses dans ledit module ; et
dans lequel au moins une partie des dimensions des pores des fibres creuses présentes dans ledit module est suffisamment petite pour ne pas permettre le passage de ladite espèce à laquelle on s'intéresse, tout en étant suffisamment grande pour permettre le passage dudit, au moins un, constituant.

2. Procédé selon la revendication 1, dans lequel ledit mât de fibres creuses est un mât monocouche de fibres creuses.

3. Procédé selon la revendication 1 ou la revendication 2, dans lequel lesdites fibres creuses ont une taille de pores d'environ 1 à environ 1000 kDa.

4. Procédé selon l'une quelconque des revendications 1 à 3, dans lequel lesdites espèces auxquelles on s'intéresse sont choisies dans le groupe constitué de cellules, de protéines et de virus.

5. Procédé selon la revendication 4, dans lequel lesdites cellules sont des cellules de mammifères.

6. Procédé selon la revendication 4, dans lequel lesdites cellules sont des cellules E. coli.

7. Procédé selon l'une quelconque des revendications précédentes, dans lequel ladite espèce à laquelle on s'intéresse est un constituant d'un lysate de E. coli.

8. Procédé selon la revendication 4, dans lequel lesdites protéines sont des protéines exprimées par recombinaison.

9. Procédé selon l'une quelconque des revendications précédentes, dans lequel ledit élément de filtre à fibres creuses a une forme sensiblement cylindrique.

10. Procédé de séparation d'une espèce à laquelle on s'intéresse d'au moins un constituant d'un mélange, ledit procédé comprenant la filtration d'un fluide contenant ladite espèce à laquelle on s'intéresse à travers un module de filtration à membrane à fibres creuses, dans lequel le module de filtration à membrane à fibres creuses comporte :
(a) une enveloppe de cartouche à filtre ; et
(b) un mât de fibres creuses de restriction d'écoulement contenu dans ladite enveloppe de cartouche à filtre, dans lequel ledit mât comporte des fibres creuses configurées sensiblement en parallèle et une matière entrelacée avec lesdites fibres creuses afin que la matière entrelacée occupe au moins environ 5% du volume vide entre lesdites fibres creuses, restreignant ainsi l'écoulement d'un filtrat entre lesdites fibres creuses et maintenant une pression sensiblement constante à travers la membrane sur toute la longueur des fibres creuses dans ledit module ; et
dans lequel au moins une partie des tailles des pores des fibres creuses présentes dans ledit module est suffisamment faible pour ne pas permettre le passage de ladite espèce à laquelle on s'intéresse, tout en étant suffisamment grande pour permettre le passage dudit, au moins un, constituant.

11. Procédé selon la revendication 10, dans lequel ladite matière occupe environ 15% à 99% du volume vide entre lesdites fibres creuses.

12. Procédé selon la revendication 10 ou la revendication 11, dans lequel lesdites fibres creuses ont une taille de pores d'environ 1 à environ 1000 kDa.

13. Procédé selon l'une quelconque des revendications 10 à 12, dans lequel lesdites espèces auxquelles on s'intéresse sont choisies dans le groupe consistant en des cellules, des protéines et des virus.

14. Procédé selon l'une quelconque des revendications précédentes, dans lequel ladite espèce à laquelle on s'intéresse est une protéine exprimée par recombinaison.

15. Procédé selon l'une quelconque des revendications précédentes, dans lequel ladite espèce à laquelle on s'intéresse est une cellule ou un constituant de cellule.

16. Procédé selon les revendications 10 ou 11, dans lequel au moins une partie des tailles des pores est comprise dans la plage d'environ 1 à 1000 kDa.

17. Procédé selon l'une quelconque des revendications précédentes, qui comprend en outre l'étape de recirculation en co-courant du filtrat sur le côté à filtrat desdites fibres creuses.

18. Procédé de réalisation d'un module de filtration à fibres creuses utile pour séparer des espèces auxquelles on s'intéresse d'un mélange, ledit procédé comprenant :
(a) l'utilisation d'un mât de fibres creuses ;
(b) la stratification physique dudit mât de fibres creuses sur un tamis de restriction d'écoulement pour former un complexe mât de fibres creuses/tamis ;
(c) la configuration dudit complexe mât de fibres creuses/tamis en une forme dans laquelle lesdites fibres creuses sont en un faisceau et ledit tamis de restriction d'écoulement occupe une partie du volume vide entre lesdites fibres creuses afin que l'écoulement d'un filtrat entre les fibres creuses soit restreint et que la pression à travers la membrane soit sensiblement constante sur toute la longueur des fibres creuses dans le module, formant ainsi un élément de filtre à fibres creuses ; et
(d) l'introduction dudit élément de filtre à fibres creuses dans une enveloppe de cartouche à filtre, ledit élément de filtre à fibres creuses étant positionné à l'intérieur de ladite enveloppe de cartouche à filtre afin de permettre la filtration d'un fluide à travers ledit élément de filtre à fibres creuses.

19. Procédé selon la revendication 18, dans lequel l'étape de configuration comprend l'enroulement dudit complexe mât de fibres creuses/tamis en une forme sensiblement cylindrique.

20. Procédé selon la revendication 18 ou la revendication 19, dans lequel ledit mât de fibres creuses est un mât monocouche.

21. Procédé selon l'une quelconque des revendications 18 à 20, dans lequel l'étape de stratification physique comprend le fait de faire adhérer de façon permanente ledit mât de fibres creuses audit tamis.
